# EUROPEAN PATENT SPECIFICATION

(11) **EP 0 946 468 B1**
(45) Date of publication and mention of the grant of the patent: **23.04.2003**
(21) Application number: 97934193.0
(22) Date of filing: 14.07.1997
(51) Int. Cl.: C07C 1/02, C10G 1/10, C10G 1/00

(54) **PARTIAL OXIDATION OF WASTE PLASTIC MATERIAL**
PARTIELLE OXIDATION VON ABFALLPLASTIKMATERIAL
OXYDATION PARTIELLE DE DECHETS DE MATIERES PLASTIQUES

(30) Priority: 17.07.1996 US 21878 P; 17.07.1996 US 21879 P; 17.07.1996 US 21885 P; 03.07.1997 US 888144
(43) Date of publication of application: 06.10.1999
(73) Proprietor: TEXACO DEVELOPMENT CORPORATION, White Plains, New York 10650 (US)
(72) Inventor: WINTER, John, Duckett, Yorba Linda, CA 92886 (US); BRICKHOUSE, Paul, Ellis, Houston, TX 77079 (US); TYREE, Ronald, Frederick, Houston, TX 77083 (US); STEVENSON, John, Saunders, Yorba Linda, CA 92887 (US); MAYOTTE, Gregory, Joseph, Wappingers Falls, NY 12590-3723 (US); KASSMAN, Jerrold, Samuel, League City, TX 77573 (US); KLOCK, Byron, Von, Beaumont, TX 77708 (US)
(74) Representative: Eddowes, Simon
(86) International application number: US9712480
(87) International publication number: WO98002401

(56) References cited:
- US-A- 5 445 659
- US-A- 5 457 250
- US-A- 5 534 040

## Description

### BACKGROUND OF THE INVENTION

### 1. Field of the Invention

This invention relates to an environmentally safe process for converting waste plastic materials to valuable gas products and environmentally nontoxic slag, and more particularly to a minimum liquid discharge integrated liquefaction and gasification process for converting bulk halogen-containing waste plastic materials into a liquid hydrocarbonaceous feedstock that can be used in a partial oxidation reactor to produce a synthesis gas that is primarily carbon monoxide and hydrogen, and a non-leachable environmentally nontoxic slag.

### 2. Description of the Prior Art

Waste plastic materials, especially halogen-containing waste plastic materials, and particularly those with a high chloride content present especially difficult disposal problems, since these materials are subject to increasingly stringent environmental restrictions against dumping or landfill disposal. Burning the waste plastic materials is feasible only if conducted in accordance with strict environmental restrictions against atmospheric disposal of chloride-containing gases and toxic particulate by-products.

Canadian Patent Application No. 2,130,019 to Gerhardus et al. (equivalent to US 5 457 250) relates to a process for thermally cracking waste plastic materials which then undergo partial oxidation to produce synthesis gas. However, it is necessary to dehalogenate the waste plastic materials prior to partial oxidation because of severe corrosion problems that can occur without dehalogenation when halide-containing vapors accompanying the syngas products are cooled and condensed. The halide vapors, primarily in the form of hydrogen chloride are condensed from the gaseous degradation products which occur during liquefaction of the waste plastic materials. Dehalogenation, especially dechlorination prior to partial oxidation is an important concern because the Canadian patent relies upon radiant cooling of the partial oxidation gasification products. Therefore, the presence of hydrogen chloride or halide vapors would present severe corrosion problems in the equipment used in the process of the Canadian patent.

German Patent Application DE 4412360A1 to Rabe et al. discloses a process for recycling mixed and contaminated waste plastic materials in a gasification reaction to produce carbon monoxide and hydrogen. The German process utilizes liquefaction and gasification steps but does not address the problem of dealing with halogen-containing waste plastic materials.

US 5534040 discloses a process for up-grading plastic material containing inorganic filler or reinforcement material for use as feedstock in a partial oxidation gas generator for the production of raw synthesis gas, fuel gas or reducing gas. The plastic material is granulated and partially liquified by heating in a closed autoclave while the plastic material is in contact with a pumpable hydrocarbonaceous liquid solvent. A pumpable slurry is thereby produced and, after removal of any separated inorganic material, the remainder of the slurry is reacted by partial oxidation to produce raw synthesis gas, fuel gas or reducing gas.

US 5445659 discloses a process for converting scrap plastic material into a high quality transportation fuel. The plastic material is granulated and mixed with granulated solid carbonaceous fuel and liquid hydrocarbonaceous solvent to produce a plastic containing sludge. The plastic containing sludge is liquified in a closed autoclave while in contact with hydrogen gas. The pumpable slurry from the autoclave is heated and fractionated in a fractionation zone to produce a hydrocarbonaceous distillate which may be used for transportation fuel. A bottoms stream from the fractionation zone comprising liquid hydrocarbonaceous material and inorganic material is reacted by partial oxidation to produce non toxic slag and raw synthesis gas, fuel gas or reducing gas which may be purified in a gas purification zone.

As used herein, a partial oxidation reactor can also be referred to as a "partial oxidation gasifier," or simply a "gasifier," and these terms are often used equivalently and interchangeably. The partial oxidation reactors that are used in this invention are also commonly referred to as "quench gasifiers."

Reaction temperatures for partial oxidation typically range from 900°C to 2,000°C, preferably from 1,200°C to 1,600°C. Pressures typically range from 0.1 to 25.3 MPa (1 to 250 atmospheres) preferably from 0.5 to 20.3 MPa (5 to 200 atmospheres) most preferably 2 to 8.1 MPa (20 to 80 atmospheres).

Partial oxidation reactors are disclosed in U.S. Patent No. 4,823,741 to Davis et al., U.S. Patent No. 4,889,540 to Segerstrom et al., U.S. Patent Nos. 4,959,080 and 4,979,964, both to Sternling, U.S. Patent No. 5,281,243 to Leininger, and U.S. Patent Nos. 5,554,202 and 5,545,238 both to Brooker et al.

### SUMMARY OF THE INVENTION

The present invention relates to a liquefaction process for converting a halogen-containing bulk waste plastic material into the liquid hydrocarbonaceous feedstock for a partial oxidation reaction to produce a synthesis gas, comprising:
(a) melting a halogen-containing bulk waste plastic material having an average particle size not exceeding 0.46 metres (18 inches) in a melting zone in direct contact with a hot oil heating medium at a minimum temperature necessary to melt the waste plastic material and in the presence of water to suppress the formation of halohydrocarbon vapors to produce a molten viscous mixture of the halogen-containing waste plastic material with the hot oil, and a first offgas;
(b) thermally cracking the molten viscous oil/plastic mixture in a heating zone at a minimum temperature necessary to thermally crack the molten viscous plastic material to produce a halogen-containing cracked oil composition of reduced viscosity;
(c) removing the cracked oil from the heating zone and separating it so that a portion thereof is recycled to the melting zone to serve as the hot oil melting medium and the remainder is passed to the reaction zone of a quench gasifier;
(d) partially oxidizing the cracked oil in the reaction zone of a quench gasifier wherein the cracked oil serves as the primary hydrocarbonaceous reactant in a non-catalytic partial oxidation reaction to produce a synthesis gas containing hydrocarbon halides;
(e) quenching the synthesis gas in the quench zone of the gasifier, wherein the synthesis gas is contacted with a quench water containing neutralizing agent to neutralize the hydrogen halides in the synthesis gas and thereby form condensed halide salts which are separated and recovered from the quench water, and a halogen-free synthesis gas.

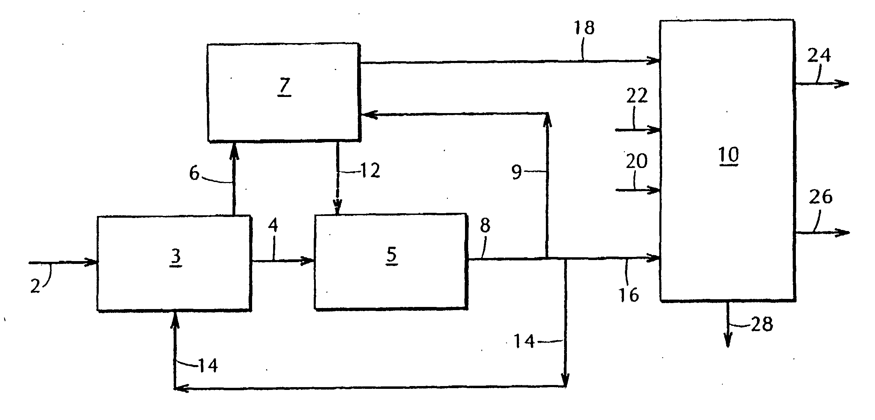

### BRIEF DESCRIPTION OF THE DRAWING

The accompanying drawing is a simplified diagrammatic representation of the liquefaction and partial oxidation stages of the process.

### DESCRIPTION OF THE PREFERRED EMBODIMENTS

The present invention is an improved environmentally safe liquefaction process for converting waste plastic materials having a high or variable halogen content into a hydrocarbonaceous feedstock for a partial oxidation reaction in a quench gasifier to produce a synthesis gas or "syngas" which primarily comprises carbon monoxide and hydrogen.

An important advantage of the liquefaction process is that it can convert bulk waste plastic material having high halogen content, into a thermally cracked oil with a minimal amount of particle size reduction, or halogen removal prior to liquefaction. The cracked oil that is produced from the waste plastic material during liquefaction serves as the primary source of the hydrocarbonaceous reactant in the partial oxidation reaction to produce synthesis gas.

The products of liquefaction include a thermally cracked oil which does not vaporize at the melting and cracking temperatures during liquefaction. The thermally cracked oil is also referred to as a "heavy oil." Liquefaction also produces condensible vapors, referred to as "condensed gases" or "condensates," and non-condensible vapors which do not condense under the cooling conditions of the liquefaction process.

It has been discovered that the wide variations in chemical composition of the waste plastic material are not reflected in the chemical composition of the thermally cracked oil. Thus, the chemical composition of the thermally cracked oil or the heavy oil is significantly less variable, than the waste plastic material, whereas the chemical composition of the condensates and/or the noncondensible gases is more consistent with that of the waste plastic feed.

This is a significant factor because the thermally cracked oil constitutes 70 weight % to 80 weight % of the gasifier feedstock. Therefore, astable composition is important. The variation in chemical composition of the condensates can be attenuated by blending. The composition variation in the noncondensible gases can be dealt with by using these gases as a fuel for the heating unit employed in the liquefaction process.

The inventive process is capable of partially oxidizing the liquefied halogen-containing waste plastic materials into useful synthesis gas with minimal liquid discharge and is environmentally nontoxic. By "environmentally nontoxic" is meant that substantially no toxic gases, liquids or solids are released to the environment. The process is designed to self-contain and not release any toxic vapors, liquids or solids. Thus, no hazardous vapors, liquids or particulate solids enter the environment. The slag byproduct is vitreous and non-leachable, and can be used as a landfill or in construction.

The term "vitreous slag" refers to a slag which chemically or physically binds elements and/or compounds which can be harmful to the environment in their free state. These bound elements and/or compounds are resistant to leaching from the slag. Consequently the vitreous slag produced herein is an environmentally nontoxic slag.

The particle size reduction of bulk waste plastic in conventional processes prior to liquefaction is perhaps one of the most expensive aspects of a waste plastic recovery operation. A principal advantage of the inventive process is that only minimal size reduction of the scrap plastic is needed. Size reduction to an average particle size of 0.46 metres (18 inches) is all that is needed to feed the waste plastic material to the liquefaction step. However, reducing the particle size to an average particle diameter of 152 mm (6 inches) and preferably 51mm (2 inches) is desirable to facilitate magnetic removal of metal contaminants or components contained in the scrap plastic. These particulate sizes denote bulk particulate waste plastic materials that have been subjected to minimal particle size reduction and which can be conveniently transported, such as by pumping the bulk particulate waste plastic materials to a liquefaction vessel.

In contrast, conventional liquefaction processes require particulate size reduction of the waste plastic to less than 1 centimeter. This generally requires expensive grinding and other equipment and special conditions to prevent melting and agglomeration of the plastic from the mechanical heat generated by such grinding operations.

Therefore, the inventive process can be characterized as "robust" because of its powerful capacity to liquefy and partially oxidize waste plastic materials with a minimal amount of size reduction. This is an important feature and benefit of the invention.

Another advantage of the inventive process is that the heat necessary to melt and crack the waste plastic material to form a hot liquid or oil, is substantially completely supplied by heat values recovered from the operational steps of the process. Therefore, the process can also be characterized as being substantially or completely autogenous.

The prior art conditions for dehalogenation are much more limiting than the simple melting, viscosity reduction and cracking operations conducted in accordance with the present invention. This is because the prior art processes operate to yield a liquid product with less than 200 parts per million (ppm) halide, preferably less than 50 ppm. In the present invention, the dehalogenation that occurs during liquefaction is incidental, and not needed, nor even particularly desirable. The prior art processes crack and operate to dehalogenate whereas the present invention cracks and operates primarily to lower the viscosity of the cracked oil formed from the waste plastic material.

Conventional dehalogenation produces a halogen-acid stream contaminated with organic materials such as organic acids, aromatics, esters, aldehydes, glycols, and the like. This halogen-acid stream is suitable only for incineration to recover pure halogen acids or salts, and is not salable as an acid because of the organic fraction contained therein. It is also not useful as a fuel or organic feedstock because the halogen content is corrosive to combustion equipment and requires stack gas scrubbing to prevent halogen-acid emissions, such as HCl. If used as an organic feedstock the halogen content can poison the catalyst and contaminate the product. Apart from the present invention, the acid stream from dehalogenation of plastics must be incinerated to yield an organic-free acid.

The waste plastic material used in the inventive process can be derived from thermoplastic or thermosetting plastics that have been used in the packaging, structural, building, electrical and textile industries. Most often these materials are obsolete or waste plastics that are no longer desirable and have been used in the manufacture of articles for daily use in the home or industry, such as containers, packaging materials, household devices, sporting equipment and toys. Waste plastic materials can also be derived from faulty manufacturing batches and unutilizable remains and residue from the production and processing of various plastic articles. Thus, plastic wastes can be simply characterized as post-consumer waste or obsolete plastic material which cannot be regenerated or economically reused. All plastics found in household waste can be tolerated in the process.

Preferably the halogen content of the waste plastic material varies from 0.5 weight % to 10 weight %. Waste plastic materials which can be used in the present invention include polyolefins, vinyl resins such as poly(vinyl chloride), poly(vinyl acetate) and poly(vinyl alcohol). In addition, polystyrenes, polycarbonates, poly(methylene oxides), polyacrylates, polyurethanes, polyamides, polyesters and hardened epoxide resins can also be used.

Referring to the FIGURE, halogen-containing bulk waste plastic materials 2 are fed to a melting vessel 3 where the waste plastic material 2 comes into direct contact with hot oil melting medium 14 under substantially oxygen-free conditions and at the minimum temperature necessary to melt the waste plastic to form a molten viscous mixture 4 comprising the halogen-containing waste plastic material 2 and the hot oil 14. The melting temperature of the bulk waste plastic is maintained as low as possible to minimize production of offgases 6 which vaporize from the waste plastic material during the melting step and comprise hydrogen halides, light hydrocarbons, halohydrocarbons such as methyl chloride and ethyl chloride, and carbon dioxide and water vapor. The most common halogen-containing compound is hydrogen chloride which is primarily produced from waste polyvinylchloride. The preferred melting temperature range is 110°C to 375°C.

Preferably the melting step is conducted is the absence of a catalyst. The offgas vapor stream 6 generated from the waste plastic material during the melting step is passed to a cooler 7. The molten viscous mixture 4 of melted plastic in hot oil is passed to heater 5 which operates at the minimum temperature necessary to thermally crack the molten viscous plastic mixture 4 into a lower boiling point, lower molecular weight halogen-containing hot oil composition 8 with reduced viscosity. The preferred viscosity is less than 3 Pa.s (3,000 centipoise (cp)), preferably 1 Pa.s (1,000 cp) or less, wherein the viscosity is measured at the exit temperature of heater 5, which varies from 350°C to 430°C, and is also the operative temperature range for the thermal cracking operation. Operating the heater 5 at this temperature minimizes the amount of offgas vapor stream 9 which generally comprises hydrogen halides, halohydrocarbons, light hydrocarbons and carbon dioxide.

Substantially no water vapor is produced in offgas stream 9 which is separated from hot cracked oil 8. The offgas vapor stream 9 and the offgas vapor stream 6 enter the cooler 7 wherein they undergo condensation at a temperature of 20°C to 70°C, to form a condensed gas stream 18 comprising a water miscible condensate, a non-water miscible hydrocarbon condensate and any condensed halides. If desired, the non-water miscible hydrocarbon condensate or a portion thereof can be separated, purified and sold commercially. A non-condensed organic gas stream 12 is also produced which serves as a fuel for heater 5. A portion 14 of hot cracked oil stream 8 is recycled to the melting vessel 3 to serve as the melting medium.

Typically, the hot oil stream 14 is recycled to the melting vessel 3 at a weight ratio of hot oil stream 14 to bulk waste plastic materials 2 of 1:1 to 6:1, respectively.

It has also been found that by introducing H₂O into the melting vessel 3 and/or the heater 5, the formation of gaseous halohydrocarbon compounds and other polar compounds such as acetone' in the offgas streams 6 and 9 can be suppressed or minimized. Chloromethane or methyl chloride, CH₃Cl, is the most volatile of the halohydrocarbonous formed from the liquefaction of waste plastic materials, and is most likely to contribute to the problems caused by the chlorine content of non-condensed fuel gas 12 entering the process heater 5.

To suppress the formation of gaseous halohydrocarbon compounds, H₂O preferably in the form of steam can be introduced into the molten oil/plastic mixture 4, and/or the cracked oil stream 8, and/or the recycle hot oil stream 14, and/or directly into melting vessel 3. In addition, the bulk waste plastic material 2 can be contacted with H₂O as it is introduced into the heating vessel 3.

Another benefit of introducing steam or water to the liquefaction process is that the steam can condense a substantial amount of hydrogen chloride (HCl) vapor. The process water condenses halogen acid vapors in offgas streams 6 and 9, thus preventing or minimizing the halogen acid vapors from becoming part of the non-condensible gas stream 12 that enters the heater 5. However the amount of H₂O must be carefully monitored for optimum results. Increasing the amount of water increases the load on the cooler/condenser 12. It has been found that 5 weight % to 15 weight % water in the waste plastic feed to melting vessel 2 or introduced into the reactor section of the gasifier 10 represents a good balance between acid content in noncondensible gases and the thermal load on the coolers.

The water content of the waste plastic materials 2 is generally sufficient to supply the necessary amount of steam to supress the formation of gaseous halohydrocarbon compounds. However, the presence of halohydrocarbons in the non-condensed gas stream 12 would indicate the need for additional steam on the order of 5 weight % H₂O to 10 weight % H₂O, based on the total weight of the waste plastic feed.

It has been found that 10 weight % H₂O in the form of steam, based on the total weight of the waste plastic feed, added directly to the molten oil/plastic mixture fed to the heater resulted in an 86% reduction of chloromethane in the offgas.

The condensed gas stream 18 containing any remaining condensed halides is fed to the gasifier 10 for the partial oxidation reaction. During the partial oxidation reaction, a synthesis gas is produced comprising carbon monoxide, hydrogen, carbon dioxide, water vapor and gaseous halides HX, where X can be chlorine, fluorine, bromine or iodine.

The non-condensed gas stream 12 from the cooler 7 that enters the heater 5 serves as a fuel therein to increase the temperature to the level needed to thermally crack the viscous molten plastic 4 into the cracked hot oil stream 8. The cracking temperature varies from 360°C to 430°C. A portion of the cracked hot oil stream 8 exiting the heater 5 is divided into streams 14 and 16 after the offgas stream 9 is separated therefrom. Hot cracked oil stream 14 is recycled to the melting vessel 3 to serve as the hot oil melting medium used to directly contact and melt the waste plastic material 2 entering melting vessel 3.

During startup, used motor oil, or any low volatility oil, or cracked oil retained from the process, can be used as the melting medium. However, once the process has become operational, the recycle stream 14 of hot cracked oil furnishes substantially or completely the entire melting medium needed to melt the halogen-containing bulk waste plastic materials 2 in the melting vessel 3.

Hot cracked oil stream 16 enters the gasifier 10 with condensed gas stream 18 to serve as the complete or substantially complete hydrocarbonaceous reactant for the partial oxidation reaction. Cracked oil stream 16 contains the remaining unvaporized halogen content of the waste plastic material, which can vary from .01 weight % to 2 weight % of oil stream 16. Oxygen or an oxygen-containing gas stream 22, such as air is fed into the reaction zone (not shown) of the quench gasifier 10 to serve as the oxidizing agent for the partial oxidation reaction.

A low heating value material 20 can be used as a temperature moderator to control the temperature in the reaction zone of the gasifier. The low heating value material 20 is passed into the reaction zone of the gasifier in amounts sufficient to control or moderate the temperature in the reaction zone to 1200°C to 1600°C. The temperature moderator can be water, ash, inert gases or mixtures thereof.

The partial oxidation reaction conducted in the gasifier 10 with the cracked oil 16, condensed gases 18, and the oxidizing agent 22 produces a synthesis gas or "syngas" comprising primarily carbon monoxide and hydrogen, and smaller amounts of carbon dioxide, water vapor, hydrogen sulfide, carbonylsulfide, hydrogen halides and methane. A molten slag byproduct is also produced.

The syngas and the slag are passed into the quench zone (not shown) of the gasifier 10 and contacted with water, referred to as "quench water." The scrubbed syngas exits the quench zone of the gasifier 10 as syngas stream 24 and is purified for further use. The slag 28 exits the quench zone of the gasifier 10 and is in a vitreous, non-leachable state and is environmentally nontoxic. The slag 28 can be used as a building material, for road fill or other purpose. The hydrogen halide gases which are produced in the reaction zone of the gasifier are condensed to form acid halides which are neutralized in the quench zone to form halide salts, such as NH₄Cl, NaCl, CaCl₂, MgCl₂, or any other equivalent halide salt, with another halogen substituted for chlorine.

A portion 26 of the quench water from the quench zone of the gasifier 10 is continuously removed and is commonly referred to as the "blowdown". The blowdown stream 26 contains any remaining finely divided residual particulate solid material and the condensed halide salts. The amount of blowdown quench water 26 that is continuously removed from the quench zone of the gasifier is based on the halide content and the amount of residual solids contained in the quench water. The halide content and the amount of finely divided residual solids is periodically measured from samples or by a process analyzer. The rate of quench water 26 removed, also referred to as "the blowdown rate" is set to maintain the halide salt concentration well below its saturation concentration under all operating conditions in the quench water system. Typically, these salt concentrations vary from 1000 ppm to 20 weight % of the quench water system. The rate of removal of the quench water is coordinated and regulated with the quench water supplied to the quench zone to maintain a constant and steady supply of quench water in the quench zone.

### EXAMPLE 1

A mixture of waste plastic materials is heated to 750°F (399°C) and melted in a stirred batch reactor at 0.1 MPa (one atmosphere) and held at this temperature for 30 minutes, to yield 80 weight % oil, 15 weight % condensible vapor, and 5 weight % noncondensible vapor (at 32°C (90°F) and 0.1 MPa (1 atmosphere)). The viscosity of the product oil is about 300 centipoise at 316°C (600°F). At 800°F (427°C) and the same time and pressure conditions, i.e., 0.1 MPa (1 atmosphere) and 30 minutes, the gas yield increases to 20 weight % and is in excess of the fuel needed for the liquefaction stage, and unsuitable for gasification, because it would be economically unfeasible to compress the excess gas and put it in the gasifier. Therefore the excess gas would be usable only if capable of being blended with associated on-site fuel gas usage.

### EXAMPLE 2

Several tons of post-consumer waste plastic materials are added over a period of 5 days to a melt tank wherein the initial start-up melting medium is used motor oil, until replaced by the continuous circulation of the mixture of cracked oil and plastics to a fired process heater and back to the melting tank as in the FIGURE. The melting temperature ranges from 204°C (400°F) to 371°C (700°F) at 0.1 MPa (one atmosphere) pressure. The entire molten mixture is heated to 780°F (416°C) in the fired process heater and returned to the melt tank. Averaged over several days, the ratio of recycled oil to plastics feed exceeds 50:1. The mass of condensible vapor and noncondensible gas generated is equivalent to the mass of plastics added.

### EXAMPLE 3

Several tons of post consumer waste plastic materials are added to melt tank 3 over a period of 12 days. The mixture of oil and melted plastics leaving the melt tank is circulated through a fired heater 5 and returned to the melt tank 3 as in the FIGURE except that a portion of condensate and heavy oils are withdrawn to storage periodically to maintain levels in the process vessels. The oils are later gasified from storage. Condensible vapors and noncondensible gases are continuously removed to storage tanks. The molten mixture is heated to temperatures ranging from 650°F (343°C) to 780F (416°C) in the heater 5 prior to being recirculated to the melt tank 3 which operates between 500°F (260°C) and 680°F (360°C). The residence time in the heater 5 averages five minutes. The gas yield ranges from 10 to 20 weight percent, and the cracked oil yield ranges from 60 to 80 weight %. Almost no viscosity reduction of the plastic/cracked oil mixture is observed at cracking temperatures below 650°F (343°C). The viscosity of the cracked oil leaving the process heater ranges from 0.08 to 0.3 Pas (80 to 300 centipoise at 600°F (316°C). The plastic derived oil and condensate generated undergo partial oxidation in gasifier 10.

The gasification performance of this oil is equal to or better than other heavy oils commercially gasified in that the conversion of carbon to synthesis gas is higher at the same gasification conditions. In all of these cases, less then 20 weight % of the chlorine in the feed plastics remained in the heavy oil.

## Claims

1. A liquefaction process for converting a halogen-containing bulk waste plastic material into the liquid hydrocarbonaceous feedstock for a partial oxidation reaction to produce a synthesis gas, comprising:
(a) melting a halogen-containing bulk waste plastic material having an average particle size not exceeding 0.46 metres (18 inches) in a melting zone in direct contact with a hot oil heating medium at a minimum temperature necessary to melt the waste plastic material and in the presence of water to suppress the formation of halohydrocarbon vapors to produce a molten viscous mixture of the halogen-containing waste plastic material with the hot oil, and a first offgas;
(b) thermally cracking the molten viscous oil/plastic mixture in a heating zone at a minimum temperature necessary to thermally crack the molten viscous plastic material to produce a halogen-containing cracked oil composition of reduced viscosity;
(c) removing the cracked oil from the heating zone and separating it so that a portion thereof is recycled to the melting zone to serve as the hot oil melting medium and the remainder is passed to the reaction zone of a quench gasifier;
(d) partially oxidizing the cracked oil in the reaction zone of a quench gasifier wherein the cracked oil serves as the primary hydrocarbonaceous reactant in a non-catalytic partial oxidation reaction to produce a synthesis gas containing hydrogen halides;
(e) quenching the synthesis gas in the quench zone of the gasifier, wherein the synthesis gas is contacted with a quench water containing neutralizing agent to neutralize the hydrogen halides in the synthesis gas and thereby form condensed halide salts which are separated and recovered from the quench water, and a halogen-free synthesis gas.

2. The process of claim 1, wherein the first offgas is cooled and condensed to form a water miscible condensate, a non-water miscible condensate and a mixture of non-condensed gases.

3. The process of claim 1, wherein the halogen-containing bulk waste plastic material provides substantially the entire hydrocarbonaceous reactant for the partial oxidation reaction.

4. The process of claim 1, wherein the melting step is conducted in the absence of a catalyst.

5. The process of claim 1, wherein the halogen content of the waste plastic materials varies from 0.5 weight % to 10 weight %.

6. The process of claim 1, wherein the halogen-containing bulk waste plastic material is melted at a temperature of 110°C to 375°C.

7. The process of claim 1, wherein a low heating value material selected from the group consisting of water, ash, inert gases and mixtures thereof, is introduced into the reaction zone of the gasifier to serve as a temperature moderator.

## Patentansprüche

1. Verflüssigungsverfahren zur Umwandlung einer Menge eines halogenhaltigen Kunststoffabfallmaterials in ein flüssiges Kohlenwasserstoff-Einsatzmaterial für eine Umsetzung unter partieller Oxidation und die Herstellung von Synthesegas, umfassend
(a) Schmelzen einer Menge eines halogenhaltigen Kunststoffabfallmaterials mit einer durchschnittlichen Teilchengröße von nicht mehr als 0,46 Meter (18 Zoll) in einer Schmelzzone in direktem Kontakt mit einem heißen Öl als Heizmedium bei mindestens einer Temperatur, welche zum Schmelzen des Kunststoffabfallmaterials ausreicht, und zwar in Gegenwart von Wasser, um die Bildung von Halogenkohlenwasserstoffdämpfen zu unterdrücken und zur Herstellung einer viskosen Schmelze von einem Gemisch aus dem halogenhaltigem Kunststoffabfallmaterial und dem heißem Öl sowie von einem ersten Abgas;
(b) thermisches Cracken der viskosen Schmelze aus Öl und Kunststoff in einer Heizzone bei mindestens einer Temperatur, welche zum thermischen Cracken des viskosen geschmolzenen Kunststoffmaterials ausreicht, und Herstellen einer halogenhaltigen Cracköl-Zusammensetzung verminderter Viskosität;
(c) Abziehen und Auftrennen des Cracköls aus der Heizzone, wobei ein Teil hiervon in die Schmelzzone zurückgeführt und das heiße Öl als Schmelzmedium eingesetzt wird und der andere Teil in die Reaktionszone des Quench-Gaserzeugers geleitet wird;
(d) partielles Oxidieren des Cracköls in der Umsetzungszone des Quench-Gaserzeugers, in welchem das Cracköl in der nicht-katalytischen partiellen Oxidationsreaktion als primärer Kohlenwasserstoffreaktant eingesetzt wird und Erzeugen eines Halogenwasserstoff-haltigen Synthesegases;
(e) Kühlen des Synthesegases in der Quenchzone des Gaserzeugers, wobei das Synthesegas mit einem Quenchwasser zusammengebracht wird, das ein neutralisierendes Mittel enthält, welches die Halogenwasserstoffe in dem Synthesegas neutralisiert und hierdurch Erzeugen von kondensierten Halogensalzen, welche aus dem Quenchwasser abgetrennt und gewonnen werden, sowie eines Synthesegases frei von Halogenen.

2. Verfahren nach Anspruch 1, wobei das erste Abgas gekühlt und kondensiert wird und man ein mit Wasser mischbares Kondensat, ein nicht mit Wasser mischbares Kondensat sowie ein Gemisch von nicht kondensierten Gasen erhält.

3. Verfahren nach Anspruch 1, wobei die Menge von halogenhaltigen Kunststoffabfallmaterial im Wesentlichen den gesamten Kohlenwasserstoffreaktanten für die partielle Oxidationsreaktion stellt.

4. Verfahren nach Anspruch 1, wobei der Schmelzschritt in Abwesenheit eines Katalysators erfolgt.

5. Verfahren nach Anspruch 1, wobei der Halogengehalt des Kunststoffabfallmaterials von 0,5 Gew.% bis 10 Gew.% schwankt.

6. Verfahren nach Anspruch 1, wobei die Menge von halogenhaltigem Kunststoffabfallmaterial bei einer Temperatur von 110°C bis 375°C eingeschmolzen wird.

7. Verfahren nach Anspruch 1, wobei ein Material von geringem Heizwert, ausgewählt aus der Gruppe Wasser, Asche, Inertgase und Gemische hiervon, in die Reaktionszone des Gaserzeugers gegeben wird, wo es als Temperaturmoderator dient.

## Revendications

1. Procédé de liquéfaction pour convertir un matériau plastique de rebut en vrac contenant des halogènes en charge d'alimentation hydrocarbonée liquide pour une réaction d'oxydation partielle pour produire un gaz de synthèse, comprenant :
(a) la fusion d'un matériau plastique de rebut en vrac contenant des halogènes ayant une taille moyenne de particule ne dépassant pas 0,46 mètres (18 pouces) dans une zone de fusion en contact direct avec un milieu de chauffage à huile chaude à une température minimale nécessaire pour faire fondre le matériau plastique de rebut et en présence d'eau pour supprimer la formation de vapeurs d'hydrocarbures halogénés pour produire un mélange visqueux fondu du matériau plastique de rebut contenant des halogènes avec l'huile chaude, et un premier gaz effluent ;
(b) le craquage thermique du mélange visqueux fondu huile/plastique dans une zone de chauffage à une température minimale nécessaire pour craquer thermiquement le matériau plastique visqueux fondu pour produire une composition d'huile de craquage de viscosité réduite contenant des halogènes;
(c) l'élimination de l'huile de craquage de la zone de chauffage et sa séparation de façon qu'une partie de ladite huile soit recyclée dans la zone de fusion pour servir de milieu de fusion à huile chaude, le restant étant envoyé dans la zone de réaction d'un gazéificateur d'extinction ;
(d) l'oxydation partielle de l'huile de craquage dans la zone de réaction d'un gazéificateur à extinction dans laquelle l'huile de craquage sert de principal réactant hydrocarboné dans une réaction d'oxydation partielle non catalytique pour produire un gaz de synthèse contenant des halogénures d'hydrogène ;
(e) l'extinction du gaz de synthèse dans la zone d'extinction du gazéificateur, dans laquelle le gaz de synthèse est mis en contact avec une eau d'extinction contenant un agent neutralisant pour neutraliser les halogénures d'hydrogène dans le gaz de synthèse et ainsi former des sels halogénés condensés qui sont séparés de l'eau d'extinction et récupérés, et un gaz de synthèse sans halogènes.

2. Procédé selon la revendication 1, dans lequel le premier gaz effluent est refroidi et condensé pour former un condensat miscible dans l'eau, un condensat non miscible dans l'eau et un mélange de gaz non condensés.

3. Procédé selon la revendication 1, dans lequel le matériau plastique de rebut en vrac fournit pour l'essentiel la totalité du réactant hydrocarboné pour la réaction d'oxydation partielle.

4. Procédé selon la revendication 1, dans lequel l'étape de fusion est conduite en l'absence d'un catalyseur.

5. Procédé selon la revendication 1, dans lequel la teneur en halogène des matériaux plastiques de rebut varie de 0,5% en poids à 10% en poids.

6. Procédé selon la revendication 1, dans lequel le matériau plastique de rebut en vrac contenant des halogènes est fondu à une température de 110°C à 375°C.

7. Procédé selon la revendication 1, dans lequel un matériau de faible valeur thermique sélectionné dans le groupe composé de l'eau, de la cendre, de gaz inertes et de leurs mélanges, est introduit dans la zone de réaction du gazéificateur pour servir de modérateur de température.
